# EUROPEAN PATENT SPECIFICATION

(11) **EP 2 593 539 B1**
(45) Date of publication and mention of the grant of the patent: **09.11.2016**
(21) Application number: 10742898.9
(22) Date of filing: 15.07.2010
(51) Int. Cl.: C12N 1/20, A23C 9/123, A23C 9/13

(54) **USE OF MANGANESE FOR ENHANCING THE GROWTH OF L. CASEI IN MIXED CULTURES**
VERWENDUNG VON MANGAN FÜR VERSTÄRKTES WACHSTUM VON L.CASEI IN MISCHKULTUREN
UTILISATION DE MANGANÈSE POUR RENFORCER LA CROISSANCE DE L. CASEI DANS DES CULTURES MIXTES

(43) Date of publication of application: 22.05.2013
(73) Proprietor: Compagnie Gervais Danone, 75009 Paris (FR)
(72) Inventor: MARCHAL, Laurent, 91360 Villemoisson Sur Orge (FR); COLIN, Cyril, 92290 Chatenay Malabry (FR)
(74) Representative: Gevers & Orès
(86) International application number: PCT/IB2010/053237
(87) International publication number: WO 2012/007794

(56) References cited:
- EP-A1- 0 130 228
- EP-A1- 2 119 766
- EP-A1- 2 251 020
- EP-A1- 2 548 948
- MACLEOD, ROBERT A. AND SNELL, ESMOND E.: "Some mineral requirements of the lactic acid bacteria", JOURNAL OF BIOLOGICAL CHEMISTRY, vol. 170, 1 September 1947 (1947-09-01), pages 351-365, XP007917837,
- BRUYNEEL B ET AL: "MANGANESE AS A CONTROLLING FACTOR IN MIXED CULTURES OF LACTOBACILLUS-PLANTARUM AND ENTEROBACTERIACEAE", ANTONIE VAN LEEUWENHOEK, vol. 57, no. 2, 1990, pages 119-122, XP009146220, ISSN: 0003-6072
- RACCACH M: "Manganese and lactic acid bacteria", JOURNAL OF FOOD PROTECTION, INTERNATIONAL ASSOCIATION FOR FOOD PROTECTION, US, vol. 48, no. 10, 1 September 1985 (1985-09-01), pages 895-898, XP008110593, ISSN: 0362-028X

## Description

The invention relates to a method for enhancing the growth of bacteria from the *Lactobacillus casei* group, in mixed cultures.

The *Lactobacillus casei* group encompasses several species of lactic acid bacteria of great interest for the food industry. In particular, strains of the *Lactobacillus casei* group, belonging to the species *L. casei* subsp. *casei, L. casei* subsp. *paracasei*, and *L. casei* subsp. *rhamnosus*, have been reported to have health-promoting properties, and are used as probiotics in different food products, including in particular dairy products.

Unless otherwise specified, the terms *Lactobacillus casei* (or *L. casei)* is used hereinafter to designate any subspecies of the *Lactobacillus casei* group, and in particular any of the subspecies *casei, paracasei*, or *rhamnosus* mentioned above.

A drawback of the use of bacteria of the *Lactobacillus casei* group in the production of fermented dairy products is their slow growth in milk.

When used in pure culture, the fermentation time of *L. casei* is very long, which is a limitation in its use in industrial production.

In the manufacture of probiotic dairy products, *Lactobacillus casei* bacteria are often associated with other species of lactic acid bacteria, probiotic or not. In many cases they are combined with yogurt bacteria (*Streptococcus thermophilus* and *Lactobacillus delbrueckii* ssp. *bulgaricus* or ssp. *lactis),* which give specific organoleptic properties (such as texture and flavour) to the fermented product. Yogurt bacteria grow faster in milk than *Lactobacillus casei,* thus allowing to speed up the global fermentation process. However, the fast acidification of the medium by these yogurt bacteria limits the growth of *L. casei* in the mixed culture and therefore its population in the final product.

The inventors have now found that when manganese was added to a co-culture of *Lactobacillus casei* with other lactic acid bacteria, in particular yogurt bacteria, the growth of *L. casei* was enhanced, while no effect was observed on the other lactic acid bacteria.

An object of the invention is therefore the use of manganese for selectively enhancing the growth of bacteria from the *Lactobacillus casei* group in a mixed culture containing lactic acid bacteria not belonging to the *Lactobacillus casei* group.

More specifically, the invention provides a method for selectively enhancing the growth of bacteria from the *Lactobacillus casei* group in a mixed culture containing lactic acid bacteria not belonging to the *Lactobacillus casei* group, wherein said method comprises:
a) providing a fermentation medium containing manganese;
b) inoculating said fermentation medium with the bacteria ;
c) fermenting the inoculated medium until it reaches a desired target pH;
d) stopping the fermentation and recovering the fermented product.

The bacteria of the *Lactobacillus casei* group are selected preferably among the species *L. casei* subsp. *casei, L. casei* subsp. *paracasei, L. casei* subsp. *rhamnosus.* The lactic bacteria not belonging to the *Lactobacillus casei* group are selected preferably among *Lactobacillus delbrueckii* ssp *bulgaricus, Lactobacillus delbrueckii* ssp *lactis* and *Streptocccus thermophilus.*

The fermentation medium is preferably a dairy fermentation medium, i.e. a milk-based liquid medium. Any animal or vegetal milk source could be considered. Cow milk is however preferred. It may be for instance whole, partially or fully skimmed milk, optionally reconstituted from powdered milk. It may also consist of a milk fraction, for instance whey, or mixtures of two or more of milk fractions. Preferably, said medium will be used without supplementation other than manganese. However, it may optionally be supplemented with ingredients such as sugars, starch, thickeners, etc. provided that these ingredients do not interfere with the growth of one or more of the co-cultured strains, and provided that they are suitable for human or animal consumption.

Said medium is classically sterilized before inoculation with the *Lactobacillus* strains. Sterilization is performed by methods known in themselves, such as heat treatment.

The pH of the fermentation medium prior to inoculation with the bacteria is preferably from 5.5 to 7, preferably of about 6.4.

According to a preferred embodiment of the invention, the fermentation medium contains at least 0.1mg/l, preferably at least 0.5mg/l, and up to 100 mg/l of manganese. Generally, it will contain from about 5 to about 60 mg/l of manganese. Manganese is added in the form of a food-grade manganese salt in appropriate amount to obtain the above-mentioned final concentrations of manganese anion in the fermentation medium. Examples of food-grade manganese salts which can be used in the method of the invention include manganese chloride, manganese oxide, manganese sulfate, manganese citrate, manganese glycerophosphate, manganese gluconate, and the like. In the case of a dairy fermentation medium, the amount of manganese added will be of at least 0.05 mg, preferably of at least 0.45 mg, in order to take in account the amount of manganese naturally present in milk (which is of about 0,05 + /- 0,02 mg/l).

The manganese salt can be added to the fermentation medium before inoculation with the bacteria, or simultaneously with said inoculation. Advantageously, it can also be added to the *L. casei* inoculum during its preparation, for instance it can be added after concentration of the propagated bacteria, and before freezing the concentrate. In this case the amount of manganese in the inoculum will be generally of from 0,5 mg/ml to 4,5.mg/ml.

Generally, the bacteria of the *Lactobacillus casei* group are inoculated in the medium in a quantity of at least 10⁴ CFU/ml, preferably of from 5 x 10⁴ to 1 x 10¹⁰ CFU/ml, more preferably of from 10⁶ to 10⁸ CFU/ml, and still more preferably of about 2.10⁷ CFU/ml.

The lactic acid bacteria not belonging to the *Lactobacillus casei* group are inoculated in the medium in a quantity of from 10³ CFU/ml to 10⁹ CFU/ml, preferably of from 10⁵ to 10⁸ CFU/ml, more preferably of from 10⁶ to 10⁷ CFU/ml, and still more preferably of about 5.10⁶ CFU/ml.

More specifically, in the case of co-culture *of L. casei* with yogurt bacteria, *Streptococcus thermophilus* are inoculated in the medium in a quantity of from 10⁵ CFU/ml to 10⁸ CFU/ml, preferably of from 10⁶ to 10⁷ CFU/ml, more preferably of about 5.10⁶ CFU/ml, and *Lactobacillus delbrueckii* ssp. *bulgaricus* or *Lactobacillus delbrueckii* ssp *lactis* ssp. *lactis* are inoculated in the medium in a quantity of from 10⁴ CFU/ml to 10⁸ CFU/ml, preferably of from 10⁵ to 10⁷ CFU/ml, more preferably of about 10⁶ CFU/ml.

After inoculation of the dairy medium, fermentation is conducted under the usual conditions suitable for growth of the inoculated bacteria.

The fermentation temperature will be generally of from 15°C to 45°C, preferably of from 25°C to 43°C, most preferably of about 36°C.

The fermentation is stopped when the fermentation medium reaches the desired target pH and/or when the *Lactobacillus casei* population reaches the desired target value.

Generally, the target pH will be of from 3.7 to 4.9, preferably of from 4 to 4.8, most preferably of about 4.6.

The target value for the *Lactobacillus casei* final population is generally of at least 1.1 times, preferably of at least 1.3 times and usually up to 1.5 times the final population obtained when the same *Lactobacillus casei* bacteria are cultured under the same conditions without Mn.

Generally, the fermentation is stopped when the *L. casei* population is of at least 10⁷ CFU/ml, preferably of at least 10⁷ to 10¹⁰ CFU/ml.

The invention also relates to a fermented dairy product obtainable by the process of the invention. This product usually contains, in addition to the bacteria used for the fermentation, the fermented dairy medium, containing the added manganese salt. This fermented dairy product can be used as such, in particular as a food product, or a nutritional supplement.

It can also be added to a food product, in particular a dairy product such as a yogurt, or to a nutritional, pharmaceutical, or cosmetic composition. The food composition, as well as the nutritional, pharmaceutical, or cosmetic compositions comprising said fermented dairy product are also part of the invention.

The present invention is further illustrated by the additional description which follows, which refers to non-limiting examples of the implementation of the process of the invention.

### EXAMPLE 1: SELECTIVE ENHANCEMENT BY MANGANESE OF GROWTH OF L. CASEI SSP PARACASEI CO-CULTURED WITH YOGOURT BACTERIA.

A dairy fermentation medium was prepared by mixing skimmed milk powder (30 g) with distilled water (920 g), sucrose (50g), with (medium A) or without (medium B) addition of monohydrate manganese sulfate at a final concentration of 61,44 mg/l, corresponding to 20mg/l of manganese cation. After 1 h rehydratation, the mix was sterilized by autoclaving for 15 mn at 115°C.

Medium A and medium B are inoculated with *Lactobacillus casei* subsp. *paracasei* DN-114 121 (3.80 x 10⁷ CFU/ml), and a yogurt ferment (DN 542 142) consisting of: *Streptococcus thermophilus* DN-001 640 (10⁶ CFU/ml), *Streptococcus thermophilus* DN-001 336 (2. 10⁶ CFU/ml), *Streptococcus thermophilus* DN-001 236 (7. 10⁵ CFU/ml) and *Lactobacillus bulgaricus* DN-100 290 (10⁵ CFU/ml).

Fermentation was conducted at 36°C, and monitored by measuring the decrease of pH in the culture medium. Fermentation was stopped by transferring the preparation at 4°C when a target pH of 4,6 was reached.

The target pH was reached after 6,1 hours fermentation in the case of medium A, and 6,6 hours fermentation in the case of medium B.

The population of *Lactobacillus casei* subsp. *paracasei,* was evaluated at J+3 by cell count analysis. Cell count analysis was performed by serial dilutions of the fermented medium with tryptone salt solution, and plating on Petri dishes with appropriate agar medium: MRS Agar containing 1.5 g/l of BactoOXGALL (DIFCO) for *Lactobacillus casei.*

Petri dishes were incubated at 37°C during 96 H in anaerobic conditions (5% CO₂).

The *Lactobacillus casei* population in the fermented product obtained from medium A was of 1.2 x 10⁸ CFU/ml.

The *Lactobacillus casei* population in the fermented product obtained from medium B was of 8.8 x 10⁷ CFU/ml.

The populations of *Streptococcus thermophilus* and *Lactobacillus bulgaricus* were not evaluated by cell counts, but by the evaluation of the acidification kinetics. It is well known that *Streptococcus thermophilus* and *Lactobacillus bulgaricus* grow faster in milk than *L. casei* and their lactic acid productions are directly correlated to their growth. Also, in the present invention, the monitoring of acidification corresponds to the monitoring of *Streptococcus thermophilus* and *Lactobacillus bulgaricus* growth.

The experimentations were performed again with *Lactobacillus casei* subsp. *paracasei* DN-114 121, on the same dairy fermentation medium as above, with or without monohydrate manganese sulfate at a final concentration of 61,44mg/L, corresponding to 20mg/l of manganese cation.

Two yogurt ferments were tested in combination with *Lactobacillus casei:* DN 542 142 described above, and DN 543 043, consisting of a strain of *Streptococcus thermophilus* (DN-.001 171) and a strain of *Lactobacillus bulgaricus* (DN-100 182).

*Lactobacillus casei* subsp. *paracasei* DN-114 121 was inoculated at 3.8 10⁷ CFU/ml.

DN 542 142 was inoculated at 10⁶ CFU/ml for *Streptococcus thermophilus* DN-001 640 ; 2.10⁶ CFU/ml for *Streptococcus thermophilus* DN-001 336; 7.10⁵ CFU/ml for *Streptococcus thermophilus* DN-001 236, and 10⁵ CFU/ml for *Lactobacillus bulgaricus* DN-100 290.

DN 543 043 was inoculated at 2.10⁶ CFU/ml for *Streptococcus thermophilus* DN-001 171; and 7.10⁴ CFU/ml for *Lactobacillus bulgaricus* DN-100 182.

Fermentation was conducted at 36°C, and monitored by measuring the decrease of pH in the culture medium. Fermentation was stopped by transferring the preparation at 4°C when a target pH of about 4.6 (in the case of the co-culture with the yogurt ferment DN 542 142), or of about 4.45 (in the case of the co-culture with the yogurt ferment DN 543 043) was reached.

The population of *Lactobacillus casei* subsp. *paracasei* was evaluated after 1 day (J+1) and 3 days (J+3) incubation at 10°C.

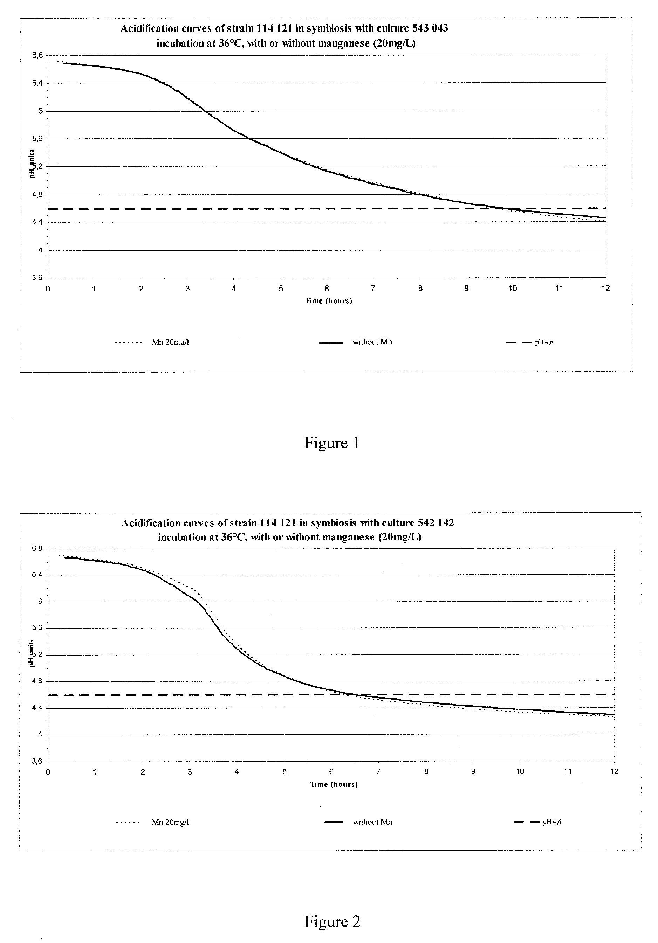

The results are illustrated by Figures 1 and 2 and Table I and II below.

**Table I**

| | End of culture | | |
|---|---|---|---|
| Tests | pH (36°C) | Time (hours) | pH J+1 (10°C) |
| 543 043 + 114 121 - Mn 20mg/L | 4,65 | 08h12 | 4,70 |
| 543 043 + 114 121 | 4,61 | 08h22 | 4,67 |
| 542 142 + 114 121 - Mn 20mg/L | 4,43 | 06h58 | 4,44 |
| 542 142 + 114 121 | 4,47 | 07h08 | 4,46 |

**Table II**

| | Count ufc/ml +10°C | | | |
|---|---|---|---|---|
| Tests | J+1 | Count. J+3 | ratio count Mn/count J+1 | ratio count Mn/count J+3 |
| 543 043 + 114 121 - Mn 20mg/L | 2,70E+08 | 3.00E+08 | 1,9 | 2,1 |
| 543 043 + 114 121 | 1,46E+08 | 1,41E+08 | | |
| 542 142 + 114 121 - Mn 20mg/L | 1,15E+08 | 1,61E+08 | 1,9 | 1,7 |
| 542 142 + 114 121 | 6,15E+07 | 9,60E+07 | | |

These results show that the addition of 20 mg/l of manganese has no effect on the acidification kinetics of the co-cultures. The time to reach the target pH is the same with or without manganese. On the other hand, the addition of 20 mg/l of manganese selectively increases the population of *Lactobacillus casei.*

In some cases the population *of L. casei* was higher at J+3 than at J+1. This can be explained by the fact that the cooling of the culture at 4°C to stop the fermentation induces some stress, resulting in a reduced ability to grow when plated on petri dishes. After a few days the bacteria recover from the stress and regain their ability to restart growth.

### EXAMPLE 2 : EFFECT OF MANGANESE ON GROWTH OF L. CASEI SSP PARACASEI OR L.CASEI SSP RHAMNOSUS CO-CULTURED WITH DIFFERENT YOGOURT FERMENTS.

The same experimentations as in Example 1 were performed with co-cultures of *Lactobacillus casei* subsp. *paracasei* DN-114 121 or *Lactobacillus casei* subsp. *rhamnosus* DN-116 010 with the yogurt ferment DN 543 043 described above, or the yogurt ferment DN 522 044, consisting of a strain of *Streptococcus thermophilus* (DN-001 143), *Streptococcus thermophilus* (DN-001 257) and a strain of *Lactobacillus lactis* (DN-111 224).

The experimentations were performed on the same dairy fermentation medium as in Example 1, with or without monohydrate manganese sulfate at final concentrations of 1,54 mg/L, 61,44 mg/L, 184,33 mg/L, corresponding to 0.5 mg/l, 20mg/l, and 60mg/l of manganese cation.

*Lactobacillus casei* subsp. *paracasei* DN-114 121 was inoculated at 3.8 10⁷ CFU/ml.

*Lactobacillus casei* subsp. *rhamnosus* DN-116 010 was inoculated at 5.8 10⁷. CFU/ml.

DN 543 043 was inoculated at 2 10⁶ CFU/ml for *Streptococcus thermophilus* DN-001 171 ; and 7 10⁴ CFU/ml for *Lactobacillus bulgaricus* DN- 100 182.

DN 522 044 was inoculated at 2 10⁶ CFU/ml for *Streptococcus thermophilus* DN-001 143 ; 10⁶ CFU/ml for *Streptococcus thermophilus* DN-001 257, 4 10⁶ CFU/ml for *Streptococcus thermophilus* DN-001 623 and 2,5 10⁵ CFU/ml for *Lactobacillus lactis* DN- 111 224.

Fermentation was conducted at 36°C, and monitored by measuring the decrease of pH in the culture medium. Fermentation was stopped by transfering the preparation at 4°C when a target pH of about 4.6 was reached.

The population of *Lactobacillus casei* subsp. *paracasei* was evaluated after 1 day (J+1) incubation at 10°C.

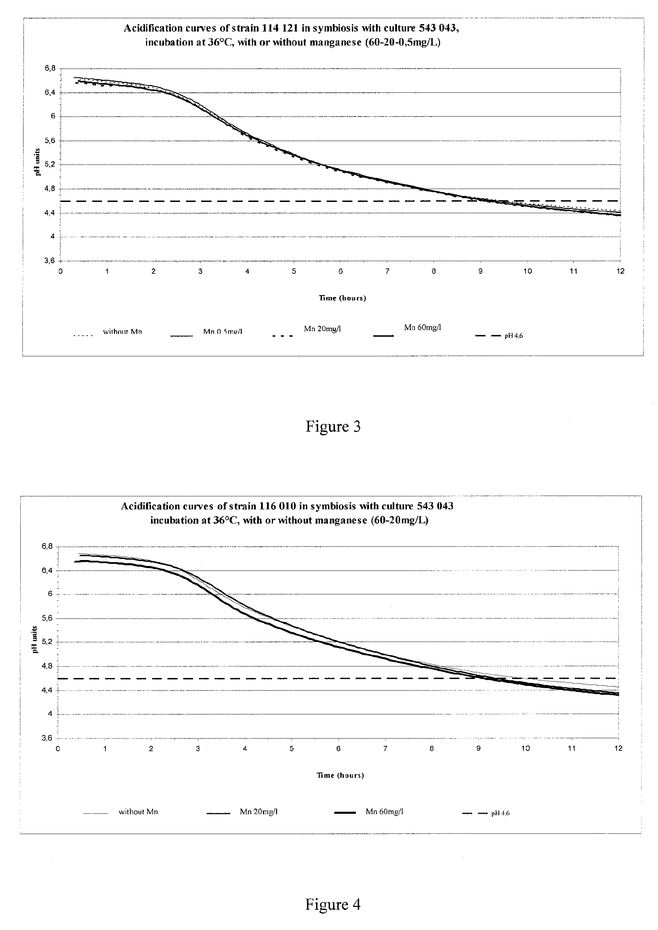

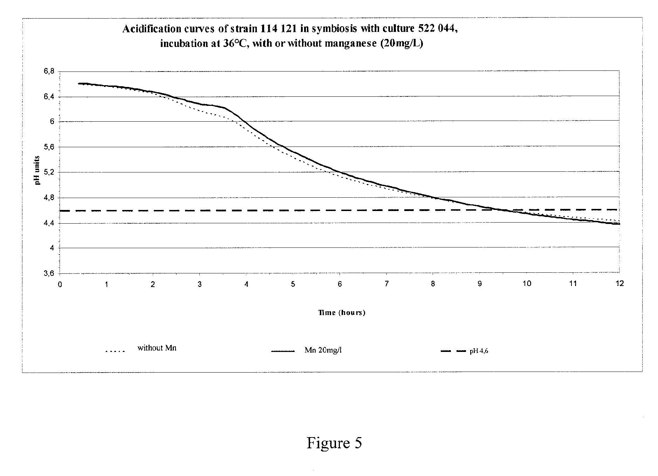

The results are illustrated by Figures 3, 4 and 5 and Tables III and IV below.

**Table III**

| | End of culture | |
|---|---|---|
| Tests | pH | Time (hours) |
| 1 - 543 043 + 114 121 0mg/L Mn | 4,60 | 09h17 |
| 2 - 543 043 + 114 121 60mg/L Mn | 4,59 | 09h14 |
| 3 - 543 043 + 114 121 20mg/L Mn | 4,58 | 09h02 |
| 4 - 543 043 + 114 121 0,5mg/L Mn | 4,60 | 09h18 |
| 5 - 543 043 + 116 010 0mg/L Mn | 4,59 | 09h50 |
| 6 - 543 043 + 116 010 20mg/L Mn | 4,60 | 09h06 |
| 7 - 543 043 + 116 010 60mg/L Mn | 4,60 | 09h04 |
| 8 - 522 044 + 114 121 0mg/L Mn | 4,60 | 09h22 |
| 9 - 522 044 + 114 121 20mg/L Mn | 4,60 | 09h20 |

**Table IV**

| | Count ufc/ml at +10°C | paracasei ou rhamnosus |
|---|---|---|
| Tests | Count J+1 paracasei ou rhamnosus | count. num. Mn/count. J+1 |
| 1 - 543 043 + 114 121 0mg/L Mn | 1,35E+08 | / |
| 2 - 543 043 + 114 121 60mg/L Mn | 3,25E+08 | 2,4 |
| 3 - 543 043 + 114 121 20mg/L Mn | 3,13E+08 | 2,3 |
| 4 - 543 043 + 114 121 0,5mg/L Mn | 2,99E+08 | 2,2 |
| 5 - 543 043 + 116 010 0mg/L Mn | 2,52E+08 | / |
| 6 - 543 043 + 116 010 20mg/L Mn | 3,20E+08 | 1,3 |
| 7 - 543 043 + 116 010 60mg/L Mn | 3,30E+08 | 1,3 |
| 8 - 522 044 + 114 121 0mg/L Mn | 1,42E+08 | / |
| 9 - 522 044 + 114 121 20mg/L Mn | 3,25E+08 | 2,3 |

These results confirm that the addition of manganese has no effect on the acidification kinetics of the co-cultures. The time to reach the target pH is the same with or without manganese. On the other hand, it selectively increases the population of the bacteria of the *L. casei* group *(Lactobacillus casei* ssp *paracasei or Lactobacillus casei* ssp *rhamnosus).*

## Claims

1. The use of manganese for selectively enhancing the growth of bacteria from the *Lactobacillus casei* group in a mixed culture containing lactic acid bacteria not belonging to the *Lactobacillus casei* group.

2. The use of claim 1, wherein the bacteria of the *Lactobacillus casei* group are selected among the species *L. casei* subsp. *casei, L. casei* subsp. *paracasei, L. casei* subsp. *rhamnosus.*

3. The use of any of claims 1 or 2, wherein the lactic bacteria not belonging to the *Lactobacillus casei* group are selected among *Lactobacillus delbrueckii* ssp *bulgaricus*, *Lactobacillus delbrueckii* ssp *lactis* and *Streptococcus thermophilus*.

4. The use of any of claims 1 to 3, wherein the bacteria are cultivated in a dairy fermentation medium.

5. A method for selectively enhancing the growth of bacteria from the *Lactobacillus casei* group in a mixed culture containing lactic acid bacteria not belonging to the *Lactobacillus casei* group, wherein said method comprises:
a) providing a fermentation medium containing manganese
b) inoculating said fermentation medium with the bacteria ;
c) fermenting the inoculated medium until it reaches a desired target pH;
d) stopping the fermentation and recovering the fermented product.

6. The method of claim 5, wherein the fermentation medium contains at least 0.1mg/l of manganese.

7. The method of any of claims 5 or 6, wherein the fermentation medium is a dairy fermentation medium, added with at least 0.05 mg/l of manganese.

8. The method of any of claims 5 to 7, wherein the bacteria of the *Lactobacillus casei* group are selected among the species *L. casei* subsp. *casei, L. casei* subsp. *paracasei, L. casei* subsp. *rhamnosus.*

9. The method of any of claims 5 to 8, wherein the lactic acid bacteria not belonging to the *Lactobacillus casei* group are selected among *Lactobacillus delbrueckii* ssp *bulgaricus*, *Lactobacillus delbrueckii* ssp *lactis* and *Streptococcus thermophilus.*

10. The method of any of claims 5 to 9, wherein the bacteria of the *Lactobacillus casei* group are inoculated in the medium in a quantity of at least 10⁴ CFU/ml, and the lactic acid bacteria not belonging to the *Lactobacillus casei* group are inoculated in the medium in a quantity of from 10³ CFU/ml to 10⁹ CFU/ml.

11. The method of any of claims 5 to 10, wherein the fermentation is conducted at a temperature of from 15°C to 45°C.

12. The method of any of claims 5 to 11, wherein the fermentation is stopped when a target pH of from 3.7 to 4.9 is reached.

13. The method of any of claims 5 to 12, wherein the final population of bacteria of the *Lactobacillus casei* group in the fermented product is of at least 1.1 times, preferably of at least 1.3 times, the final population obtained when the same *Lactobacillus casei* bacteria are cultured under the same conditions without Mn.

## Patentansprüche

1. Verwendung von Mangan zum selektiven Verstärken des Wachstums von Bakterien aus der Lactobacillus-casei-Gruppe in einer Mischkultur, die Milchsäurebakterien enthält, welche nicht zur Lactobacillus-casei-Gruppe gehören.

2. Verwendung nach Anspruch 1, wobei die Bakterien der Lactobacillus-casei-Gruppe aus den Spezies L. casei ssp. casei, L. casei ssp. paracasei, L. casei ssp. rhamnosus ausgewählt sind.

3. Verwendung nach einem der Ansprüche 1 oder 2, wobei die nicht zur Lactobacilluscasei-Gruppe gehörenden Milchsäurebakterien aus Lactobacillus delbrueckii ssp. bulgaricus, Lactobacillus delbrueckii ssp. lactis und Streptococcus thermophilus ausgewählt sind.

4. Verwendung nach einem der Ansprüche 1 bis 3, wobei die Bakterien in einem Milchfermentationsmedium kultiviert werden.

5. Verfahren zum selektiven Verstärken des Wachstums von Bakterien aus der Lactobacillus-casei-Gruppe in einer Mischkultur, die Milchsäurebakterien enthält, welche nicht zur Lactobacillus-casei-Gruppe gehören, wobei das Verfahren Folgendes umfasst:
a) Bereitstellen eines Fermentationsmediums, das Mangan enthält,
b) Impfen des Fermentationsmediums mit Bakterien,
c) Fermentieren des geimpften Mediums, bis es einen gewünschten Ziel-pH-Wert erreicht,
d) Stoppen der Fermentation und Entnehmen des fermentierten Produktes.

6. Verfahren nach Anspruch 5, wobei das Fermentationsmedium mindestens 0,1 mg/l Mangan enthält.

7. Verfahren nach einem der Ansprüche 5 oder 6, wobei das Fermentationsmedium ein Milchfermentationsmedium ist, dem mindestens 0,05 mg/l Mangan zugesetzt wurden.

8. Verfahren nach einem der Ansprüche 5 bis 7, wobei die Bakterien der Lactobacilluscasei-Gruppe aus den Spezies L. casei ssp. casei, L. casei ssp. paracasei, L. casei ssp. rhamnosus ausgewählt sind.

9. Verfahren nach einem der Ansprüche 5 bis 8, wobei die nicht zur Lactobacillus-casei-Gruppe gehörenden Milchsäurebakterien aus Lactobacillus delbrueckii ssp. bulgaricus, Lactobacillus delbrueckii ssp. lactis und Streptococcus thermophilus ausgewählt sind.

10. Verfahren nach einem der Ansprüche 5 bis 9, wobei die Bakterien der Lactobacillus-casei-Gruppe in einer Menge von mindestens 10⁴ KBE/ml in das Medium geimpft werden und die nicht zur Lactobacillus-casei-Gruppe gehörenden Milchsäurebakterien in einer Menge von 10³ KBE/ml bis 10⁹ KBE/ml in das Medium geimpft werden.

11. Verfahren nach einem der Ansprüche 5 bis 10, wobei die Fermentation bei einer Temperatur von 15 °C bis 45 °C ausgeführt wird.

12. Verfahren nach einem der Ansprüche 5 bis 11, wobei die Fermentation gestoppt wird, wenn ein Ziel-pH-Wert von 3,7 bis 4,9 erreicht ist.

13. Verfahren nach einem der Ansprüche 5 bis 12, wobei die Endpopulation von Bakterien der Lactobacillus-casei-Gruppe in dem fermentierten Produkt mindestens das 1,1-Fache, vorzugsweise mindestens das 1,3-Fache der Endpopulation beträgt, die erzielt wird, wenn die gleichen Lactobacillus-casei-Bakterien unter den gleichen Bedingungen ohne Mn kultiviert werden.

## Revendications

1. Utilisation de manganèse pour améliorer sélectivement la croissance de bactéries provenant du groupe *Lactobacillus casei* dans une culture mixte contenant des bactéries lactiques n'appartenant pas au groupe *Lactobacillus casei.*

2. Utilisation selon la revendication 1, dans laquelle les bactéries du groupe *Lactobacillus casei* sont choisies parmi les espèces *L. casei* sous-espèce *casei, L. casei* sous-espèce *paracasei, L. casei* sous-espèce *rhamnosus.*

3. Utilisation selon l'une quelconque des revendications 1 ou 2, dans laquelle les bactéries lactiques n'appartenant pas au groupe *Lactobacillus casei* sont choisies parmi *Lactobacillus delbrueckii* sous-espèce *bulgaricus, Lactobacillus delbrueckii* sous-espèce *lactis* et *Streptocccus thermophilus.*

4. Utilisation selon l'une quelconque des revendications 1 à 3, dans laquelle les bactéries sont cultivées dans un milieu de fermentation de produit laitier.

5. Procédé d'amélioration sélective de la croissance de bactéries provenant du groupe *Lactobacillus casei* dans une culture mixte contenant des bactéries lactiques n'appartenant pas au groupe *Lactobacillus casei,* dans lequel ledit procédé comprend :
a) la fourniture d'un milieu de fermentation contenant du manganèse
b) l'inoculation dudit milieu de fermentation avec les bactéries ;
c) la fermentation du milieu inoculé jusqu'à ce qu'il atteigne un pH cible souhaité ;
d) l'arrêt de la fermentation et la récupération du produit fermenté.

6. Procédé selon la revendication 5, dans lequel le milieu de fermentation contient au moins 0,1 mg/l de manganèse.

7. Procédé selon l'une quelconque des revendications 5 ou 6, dans lequel le milieu de fermentation est un milieu de fermentation de produit laitier, auquel est ajouté au moins 0,05 mg/l de manganèse.

8. Procédé selon l'une quelconque des revendications 5 à 7, dans lequel les bactéries du groupe *Lactobacillus casei* sont choisies parmi les espèces *L. casei* sous-espèce *casei, L. casei* sous-espèce *paracasei, L. casei* sous-espèce *rhamnosus.*

9. Procédé selon l'une quelconque des revendications 5 à 8, dans lequel les bactéries lactiques n'appartenant pas au groupe *Lactobacillus casei* sont choisies parmi *Lactobacillus delbrueckii* sous-espèce *bulgaricus, Lactobacillus delbrueckii* sous-espèce *lactis* et *Streptococcus thermophilus.*

10. Procédé selon l'une quelconque des revendications 5 à 9, dans lequel dans lequel les bactéries du groupe *Lactobacillus casei* sont inoculées dans le milieu en une quantité d'au moins 10⁴ UFC/ml, et les bactéries lactiques n'appartenant pas au groupe de *Lactobacillus casei* sont inoculées dans le milieu en une quantité de 10³ UFC/ml à 10⁹ UFC/ml.

11. Procédé selon l'une quelconque des revendications 5 à 10, dans lequel la fermentation est réalisée à une température de 15 °C à 45 °C.

12. Procédé selon l'une quelconque des revendications 5 à 11, dans lequel la fermentation est arrêtée quand un pH cible de 3,7 à 4,9 est atteint.

13. Procédé selon l'une quelconque des revendications 5 à 12, dans lequel la population finale de bactéries du groupe de *Lactobacillus casei* dans le produit fermenté représente au moins 1,1 fois, de préférence au moins 1,3 fois, la population finale obtenue quand les mêmes bactéries *Lactobacillus casei* sont cultivées dans les mêmes conditions sans Mn.
